(19) Europäisches Patentamt
European Patent Office
Office européen des brevets

(11) **EP 1 985 711 B1**

(12) **EUROPEAN PATENT SPECIFICATION**

(45) Date of publication and mention
of the grant of the patent:
**10.04.2013 Bulletin 2013/15**

(51) Int Cl.:
*C12P 21/02* (2006.01)          *C07K 1/30* (2006.01)
*C07K 14/02* (2006.01)

(21) Application number: **07714026.7**

(22) Date of filing: **09.02.2007**

(86) International application number:
**PCT/JP2007/052414**

(87) International publication number:
**WO 2007/094269 (23.08.2007 Gazette 2007/34)**

(54) **METHOD FOR EFFICIENT PURIFICATION OF HEPATITIS B SURFACE ANTIGEN BIONANOCAPSULE**

VERFAHREN ZUR WIRKSAMEN AUFREINIGUNG EINER HEPATITIS B OBERFLÄCHE ANTIGEN BIONANOKAPSEL

PROCÉDÉ PERMETTANT DE PURIFIER EFFICACEMENT DES BIONANOCAPSULES DE L'ANTIGÈNE DE VIRUS DE L'HÉPATITE B

(84) Designated Contracting States:
**DE FR GB**

(30) Priority: **13.02.2006 JP 2006035666**

(43) Date of publication of application:
**29.10.2008 Bulletin 2008/44**

(73) Proprietors:
• **Japan Science and Technology Agency**
**Kawaguchi-shi,**
**Saitama 332-0012 (JP)**
• **Osaka University**
**Suita-shi**
**Osaka 565-0871 (JP)**
• **Beacle Inc.**
**Okayama-shi, Okayama 701-1221 (JP)**

(72) Inventors:
• **KURODA, Shunichi**
**Suita-shi, Osaka 565-0871 (JP)**
• **MAEKAWA, Masumi**
**Aioi-shi, Hyogo 678-0001 (JP)**
• **NAGITA, Mana**
**Okayama-shi,**
**Okayama 703-8246 (JP)**

(74) Representative: **Müller-Boré & Partner**
**Patentanwälte**
**Grafinger Straße 2**
**81671 München (DE)**

(56) References cited:
EP-A- 0 135 435          EP-A- 1 491 215
WO-A1-2005/068489        GB-A- 1 511 082
JP-A- 05 076 370         JP-A- 05 252 976
JP-A- 10 101 696         JP-A- 2002 128 796
JP-A- 2003 286 296

• SANK-OK LEE, JAE-MOG SOH, BYUNG-HOON PARK BYUNG-KIL MIN: "Heat-aggregated Hepatitis B Surface Antigen and Its Effect on Immune Response" KOREAN JOURNAL OF IMMUNOLOGY, vol. 9, no. 2, 2 November 1987 (1987-11-02), pages 233-239, XP002526887
• KURODA S ET AL: "HEPATITIS B VIRUS ENVELOPE L PROTEIN PARTICLES SYNTHESIS AND ASSEMBLY IN SACCHAROMYCES CEREVISIAE, PURIFICATION AND CHARACTERIZATION" JOURNAL OF BIOLOGICAL CHEMISTRY, AMERICAN SOCIETY OF BIOLOCHEMICAL BIOLOGISTS, BIRMINGHAM, US, vol. 267, no. 3, 25 January 1992 (1992-01-25), pages 1953-1961, XP002937464 ISSN: 0021-9258
• YAMADA T. ET AL.: 'Nanoparticles for the delivery of genes and drugs to human hepatocytes' NATURE BIOTECHNOLOGY vol. 21, no. 8, 2003, pages 885 - 890, XP002980266
• YAMADA T. ET AL.: 'Physicochemical and immunological characterization of hepatitis B virus envelope particles exclusively consisting of the entire L (pre-S1 +pre-S2 + S) protein' VACCINE vol. 19, no. 23-24, 2001, pages 3154 - 3163, XP004234187

• YAMADA T. ET AL.: 'Saibo Oyobi Soshiki Tokuiteki Idenshi Donyu o Kano ni Suru Bio Nano Capsule' SEIBUTSU KOGAKU vol. 83, no. 8, 25 August 2005, pages 380 - 383, XP008128291

• KURODA S.: 'Bio Nano Capsule no Kaihatsu to Keshohin eno Oyo' FRAGANCE JOURNAL vol. 33, no. 11, 15 November 2005, pages 22 - 28

SEQUENCE LISTING

<110> Japan Science and Technology Agency
Osaka university
Beacle Inc.

<120> Method for efficient purification of bionanocapsule

<130> J 2926EU

<140> 07714026.7
<141> 2007-02-09

<150> JP 2006-35666
<151> 2006-02-13

<160> 1

<170> Patent In version 3.5

<210> 1
<211> 116
<212> PRT
<213> Staphylococcus aureus

<400> 1

```
Val Asp Asn Lys Phe Asn Lys Glu Gln Gln Asn Ala Phe Tyr Glu Ile
1                 5                 10                      15

Leu His Leu Pro Asn Leu Asn Glu Glu Gln Arg Asn Ala Phe Ile Gln
            20                  25                  30

Ser Leu Lys Asp Asp Pro Ser Gln Ser Ala Asn Leu Leu Ala Glu Ala
        35                  40                  45

Lys Lys Leu Asn Asp Ala.Gln Ala Pro Lys Val Asp Asn Lys Phe Asn
        50                  55                  60

Lys Glu Gln Gln Asn Ala Phe Tyr Glu Ile Leu His Leu Pro Asn Leu
65                  70                  75                      80

Asn Glu Glu Gln Arg Asn Ala Phe Ile Gln Ser Leu Lys Asp Asp Pro
                85                  90                  95

Ser Gln Ser Ala Asn Leu Leu Ala Glu Ala Lys Lys Leu Asn Asp Ala
            100                 105                 110

Gln Ala Pro Lys
            115
```

**Description**

The present invention relates to an efficient purification method for bio-nanocapsules composed mainly of a hepatitis B virus surface antigen (HBsAg) protein.

BACKGROUND ART

It is known that particles derived from virus proteins, such as hepatitis B virus particles, can be used as bio-nanocapsules that are usable as carriers in vivo.

For example, the inventors of the present invention have reported that by expressing a hepatitis B virus surface antigen (HBsAg) L protein in recombinant yeast cells, ellipsoidal bio-nanocapsules with a large number of L proteins embedded in yeast-derived lipid bilayer membranes can be produced (see Non-Patent Document 1). Because such particles contain no HBV genome, they do not function as viruses and are highly safe for the human body.

However, such bio-nanocapsules are produced by using non-human eukaryotic cells. Therefore, to obtain bio-nanocapsules from the cell lysate, it is necessary to remove many contaminant proteins.

For example, Patent Document 1 discloses expressing a hepatitis B virus surface antigen (HBsAg) protein in recombinant yeast cells to produce HBsAg particles (bio-nanocapsules), and then purifying the biocapsules by a combination of purification methods, such as cell disruption, cesium chloride density gradient centrifugation, dialysis, and sucrose density gradient centrifugation.

However, when purification is carried out by a density gradient centrifugation, mass production is difficult in terms of time and cost, and a more efficient method of purifying bio-nanocapsules has been desired.

Patent Document 1: Japanese Unexamined Patent Publication No. 2004-2313.

Non-Patent Document 1: J. Biol. Chem., Vol. 267, No. 3, 1953-1961, 1992.

In this context, EP 0 135 435 A2 describes a method for synthesizing a fully intramolecular disulfide bonded surface antigen protein of human Hepatitis B virus in *Saccharomyces cerevisiae.* Further, Lee, S.-O. *et al.* (Lee, S.-O. et al., Korean J. of Immunology 9(2), pp. 233-239, November 1987) describes biochemical and immunological studies on the immunogenic potency and possible changes in structure morphology of purified HBsAg after inactivation. Finally, GB 1 511 082 A describes spherical HBsAg particles which are reconstituted from HBsAg which retain the antigenicity of native HBsAg, as well as a method for preparing the same.

An object of the present invention is to purify bio-nanocapsules composed mainly of a hepatitis B virus surface antigen (HBsAg) protein using a simple, industrially applicable method.

As a result of extensive research in view of the above problem, the present inventor found that bio-nanocapsules produced used eukaryotic cells can be easily purified through thermal denaturation and precipitation of contaminant particles by heat treatment.

The inventors further found that, after heat treatment, bio-nanocapsules are further purified by chromatography methods such as Sulfate-Cellulofine chromatography and gel filtration chromatography to remove yeast- or other eukaryotic cell-derived contaminant proteins.

The present invention provides the methods for purifying bio-nanocapsules composed mainly of a hepatitis B virus surface antigen (HBsAg) protein as defined in the claims.

The bio-nanocapsule composed mainly of a hepatitis B virus surface antigen (HBsAg) protein produced used a eukaryotic cell contains contaminant particles, and eukaryotic cell-derived contaminant proteins.

Regarding these contaminants, most of the particle impurities and a considerable amount of contaminant proteins can be removed by heat treatment, whereby greatly enhanced bio-nanocapsule purity can be obtained.

Further, the bio-nanocapsules thus heat treated can be highly purified by chromatographic methods, such as affinity chromatography and/or gel filtration chromatography using a cellulofine sulfate-containing medium, a hydroxyapatite-containing medium or an anti-HBsAg antibody-bound medium, without using cesium chloride or sucrose density gradient ultracentrifugation, thereby establishing a method of purifying bio-nanocapsules on a large industrial scale.

Further, by quickly freezing and/or lyophilizing the heat-resistant bio-nanocapsule in the presence of a saccharide, the preservation stability of the bio-nanocapsule can be greatly enhanced.

FIG. 1 shows an outline of the purification method according to the present invention, and a conventional purification method.

FIG. 2 shows the results of SDS-PAGE obtained before and after the heat treatment. Lane M: molecular weight marker.

FIG. 3 shows the results of Sulfate-Cellulofine chromatography. Lane M: a molecular weight marker.

FIG. 4 shows the results of gel filtration chromatography. *1: Sulfate-Cellulofine-Recovered fractions; Lane M: molecular weight marker.

FIG. 5 shows the results of SDS-PAGE obtained throughout the process. Lane M: molecular weight marker.

FIG. 6 shows the results of preservation stability of the particles. Lane M: Molecular weight marker.

FIG. 7 shows the results of preservation stability of a lyophilizate stored at 4°C. In FIG. 7, the non-saccharide control is a bio-nanocapsule purified in the absence of sugar by the method of the present invention.

Examples of eukaryotic cells that can be used to produce the protein include yeasts, insect cells (e.g., Sf9, Sf21, and High Five cells), mammalian cells (e.g., CHO, HEK293, and COS cells), and the like. Yeasts are preferably used. Examples of yeasts include Japanese sake yeast, shochu yeast, brewer's yeast, wine yeast, baker's yeast, and other yeasts of the genera Saccharomyces, Kluyveromyces, Pichia, and the like. Brewer's yeast and baker's yeast are particularly preferable.

When an HBsAg protein or like protein capable of forming heat-resistant bio-nanocapsules is expressed in a eukaryotic cell, the protein is expressed and accumulated as a membrane protein on the endoplasmic reticulum membrane, and bio-nanocapsules are released as nanoparticles. More specifically, plasmids, which are genetic constructs capable of expressing the protein, are introduced into eukaryotic cells to transform the cells, and the obtained eukaryotic transformant cells are cultured in a medium to produce heat-resistant bio-nanocapsules. Methods for producing bio-nanocapsules are described in WO01/64930, WO03/082330, and WO03/082344. A method for preparing HBsAg is described in Vaccine, 2001 Apr. 30; 19(23-24): 3154-63, Physicochemical and immunological characterization of hepatitis B virus envelope particles exclusively consisting of the entire L (pre-S1 + pre-S2 + S) protein, Yamada T., Iwabuki H., Kanno T., Tanaka H., Kawai T., Fukuda H., Kondo A., Seno M., Tanizawa K., Kuroda S. Bio-nanocapsules can be obtained by disrupting cultured eukaryotic cells (transformants). However, the obtained bio-nanocapsules contain eukaryotic cell-derived contaminant proteins, and particle impurities that fail to function as media for introducing materials into cells.

The bio-nanocapsule is stable when heated at 50°C or more, preferably 55°C or more, and particularly 60°C or more. The bio-nanocapsule may be any nanocapsule comprising a heat-resistant protein that can be heat-treated. Thus, a nanocapsule composed mainly of hepatitis B virus surface antigen (HBsAg) protein can be used. Examples of nanocapsules composed mainly of HBsAg include nanocapsules comprising 70 to 85 wt.% of an HBsAg protein and 10 to 15 wt.% of a lipid, and those further comprising other proteins, such as hepatitis B virus core antigen (HBcAg) protein. The HBsAg protein includes natural proteins such as S protein, M protein, and L protein, and various mutants thereof.

The S protein (226 amino acid residues), which is included in HBsAg protein and is the component of S particle, has a particle-forming ability. The M protein (a constituent protein of M particle) is obtained by adding Pre-S2 composed of 55 amino acid residues to the S particle. The L protein (a constituent protein of L particle) is obtained by adding Pre-S1 composed of 108 amino acid residues (subtype y) or 119 amino acid residues (subtype d) to the M protein.

In this specification, the amino acid sequence numbering of the Pre-S1 region is based on subtype y composed of 108 amino acid residues, unless otherwise specified.

The L protein and the M protein have particle-forming abilities similar to the S protein. Therefore, the Pre-S1 and Pre-S2 regions may be optionally substituted, added, deleted and/or inserted. For example, a bio-nanocapsule that lacks a liver cell recognition ability can be produced by using a modified protein obtained by deleting a liver cell recognition site contained at positions 3 to 77 in the Pre-S1 region (subtype y). Further, the Pre-S2 region has a site capable of recognizing liver cells through albumin, and this albumin recognition site can also be deleted. In contrast, the S region (226 amino acid residues), which has a particle-forming ability, should be modified in such a manner that the particle-forming ability is not impaired. For example, when the positions 107 to 148 in the S region are deleted, the particle-forming ability is retained (J. Virol. 2002 76 (19), 10060-10063); therefore, this region may be substituted, added, deleted and/or inserted, etc. Similarly, when hydrophobic 154 to 226 residues at the C terminus are substituted, added, deleted and/or inserted, etc., the particle-forming ability can also be retained. In contrast, 8 to 26 residues (TM1) and 80 to 98 residues (TM2) in the S region are transmembrane helices (transmembrane sequences); therefore, it is desirable that this region not be mutated or that this region is deleted, added and/or substituted, etc. while maintaining the hydrophobic residues, so as to retain the transmembrane property.

In one preferable embodiment, modifications of the hepatitis B virus protein include any of the various modified proteins that have the ability to form bio-nanoparticles. Taking HBsAg protein, any number of substitutions, deletions, additions and insertions may be included in the Pre-S1 and Pre-S2 regions. In the S region, one or more, for example, 1 to 120, preferably 1 to 50, more preferably 1 to 20, still more preferably 1 to 10, and particularly 1 to 5 amino acid residues may be substituted, added, deleted or inserted. Methods for introducing mutations such as substitution, addition, deletion and insertion, in DNA encording the above protein, include gene engineering techniques such as site specific mutagenesis (Methods in Enzymology, 154, 350, 367-382 (1987); ibid., 100, 468 (1983); Nucleic Acids Res., 12, 9441 (1984)), and chemical synthesis means such as the phosphate triester method and phosphate amidite method (for example, using a DNA synthesizer) (J. Am. Chem. Soc., 89, 4801 (1967); ibid., 91, 3350 (1969); Science, 150, 178 (1968); Tetrahedron Lett., 22, 1859 (1981)). The codon for use can be selected in consideration of the codon usage in the host.

In the case of hollow bio-nanoparticles composed of a liver cell-recognizing protein, such as L protein or M protein, as a hepatitis B virus protein or modified protein thereof, introducing a cell recognition site is not necessary. On the other hand, in the case of hollow bio-nanoparticles composed of a modified protein obtained by deleting the liver cell recognition site contained at 3 to 77 amino acid residues in the Pre-S1 region, or a modified protein obtained by deleting both Pre-S1 and Pre-S2 regions, the bio-nanoparticles per se cannot recognize cells. Therefore, a cell recognition site is introduced into the bio-nanoparticles so that cells other than liver cells can be recognized and nucleic acid can be introduced into various target cells. Cell recognition sites that recognize a particular cell are preferably, for example, cell function-regulating molecules composed of polypeptides such as growth factors and cytokines; cell surface antigens; tissue-specific antigens; polypeptide molecules such as receptors, which distinguish cells and tissues; polypeptide molecules derived from viruses and microorganisms; antibodies; and sugar chains. Specific examples thereof include antibodies against EGF receptors and IL-2 receptors that are specifically expressed in cancer cells; EGF; and receptors presented by HBV. Alternative examples include proteins (e.g., ZZ tag) capable of binding an antibody Fc domain, or StreptoTag, which exhibits a biotin-like activity to present a biorecognition molecule labeled with biotin via streptavidin.

The above ZZ tag is defined as an amino acid sequence having the ability to bind to the Fc region of immunoglobulin G, and is composed of the following two repetition sequence (sequence of ZZ tag:

VDNKFNKEQQNAFYEILHLPNLNEEQRNAFIQSLKDDPSQSANLLAEAKKLNDA

QAPKVDNKFNKEQQNAFYEILHLPNLNEEQRNAFIQSLKDDPSQSANLLAEAKKL

NDAQAPK).

When the cell recognition site is a polypeptide, a hollow bio-nanoparticle that recognizes a target cell can be obtained by ligating a DNA encoding hepatitis B virus protein or a modified protein thereof to DNA encoding the cell recognition site in frame through DNA encoding a spacer peptide as needed, incorporating this into a vector or the like, and expressing it in an eukaryotic cell.

When the cell recognition site is an antibody, the desired hollow bio-nanoparticle can be obtained by ligating DNA encoding hepatitis B virus protein or a modified protein thereof to DNA encoding the ZZ tag in frame through DNA encoding a spacer peptide as needed, incorporating this into a vector or the like, expressing it in a eukaryotic cell, and mixing the resulting hollow bio-nanoparticle with an antibody capable of recognizing the target cell.

When the cell recognition site is a sugar chain, the desired hollow bio-nanoparticle can be obtained by adding (or attaching or linking) biotin to a hollow nanoparticle by chemical modification and presenting a sugar chain or the like labeled with biotin via streptavidin, etc.

The modification of the HBsAg protein may be modified proteins obtained by modifying antigenicity (modified proteins obtained by deleting/substituting a site involved in the antigenicity, such as an epitope), stability of the particle structure, cell selectivity, and the like. HBsAg (Q129R, G145R) particles described in Japanese Unexamined Patent Publication No. 2001-316298 have low antigenicity; therefore, these particles can particularly inhibit the production of antibodies against HBV and are preferably used to introduce materials, such as nucleic acids and drugs, into cells in the body.

The bio-nanocapsule comprising a modified HBsAg protein as mentioned above can be purified and preserved under the same conditions as used for bio-nanocapsules comprising an HBsAg protein such as L protein.

The method of disrupting eukaryotic cells may be any method that can disrupt the cells. Examples of such disruption methods include autolysis, enzymatic hydrolysis, acid digestion, ultrasonic disruption, homogenizers, high-pressure disruption, bead milling, grinding, freeze-thawing, and like methods. The bead milling and high-pressure disruption methods, and like methods are preferable.

The disrupted eukaryotic cell lysate is preferably subjected to heat treatment after removing water soluble materials and low-molecular-weight materials (e.g., saccharides, amino acids, oligopeptides, minerals) from the fraction containing bio-nanocapsules by dialysis.

The dialysis of the eukaryotic cell lysate can be carried out, for example, in the presence of a chelating agent such as 1mM EDTA.

When disrupting the eukaryotic cells, it is preferable to inhibit eukaryotic cell-derived (e.g., yeast-derived) protease to prevent decomposition of the heat-resistant bio-nanocapsules. For this purpose, for example, urea is preferably used as a protease inhibitor. Urea is preferable to stabilize the molecules presented on the surface of the particles during the purification process. The protease inhibitor is preferably removed by a method such as dialysis before the heat treatment process to make an efficient heat treatment.

Subsequently, if necessary, the dialyzed lysate can be heat-treated to precipitate and remove the eukaryotic cell-derived contaminant proteins. The heat treatment temperature is about 65°C to about 90°C, preferably about 65°C to about 85°C, and more preferably about 70°C to about 80°C.

When the heat treatment temperature is too low, it takes time to complete the treatment. When the temperature is too high, the antigen level is reduced, which may adversely affect the properties of the obtained bio-nanocapsules.

The heat treatment time is not particularly limited, as long as denaturation of the contaminant proteins occurs and the contaminant proteins are precipitated and removed. For example, the heat treatment time may be about 10 to about 60 minutes.

For example, Japanese Unexamined Patent Publication No. 252976/1993 describes purifying an HBsAg protein by heat treatment, in which the heat treatment temperature used is in the range of 45°C to 60°C, and preferably 50°C. Japanese Unexamined Patent Publication No. 76370/1993 describes performing a heat treatment at 30°C to 40°C, preferably at 37°C, for 1 to 6 hours to purify an HBsAg protein.

Generally, viruses are inactivated by heat treatment at 60°C. Additionally, in Chapter II, pages 371 to 398 of the 1992 WHO Global Blood Safety Initiative report "Virus Inactivation in Blood and Blood Products", it is stated that a primary method for inactivating viruses is heating at 60°C for 10 hours or more, and that most of the proteins are denatured by heat treatment in a liquid state at 60°C.

The heat treatment of the present invention is performed at 65°C or more to produce bio-nanocapsules. If the heat treatment is performed at temperatures not higher than 60°C, the contaminated proteins cannot be sufficiently separated. Thus, such a low temperature is not preferable.

After the heat treatment, the denatured contaminant proteins are precipitated and separated by centrifugation, and bio-nanocapsules can be recovered as the supernatant. The centrifugation conditions may be, for example, centrifugation at a temperature of about 4°C to about 8°C at 34,780 x g for 20 to 60 minutes.

Subsequently, the obtained supernatant-derived bio-nanocapsule particles are purified by affinity chromatography and/or gel filtration chromatography.

Preferable examples of affinity resins include resins having a sulfate group (sulfate esters), such as Sulfate-Cellulofine, Heparin-Cellulofine, and Heparin Sepharose CL6B (Pharmacia Corp.), hydroxyapatite resins, and anti-HBsAg antibody-bound resins, the anti-HBsAg antibody being produced by using HBsAg particles as an antigen. Affinity chromatography and gel filtration chromatography can be performed according to conventional methods.

The bio-nanocapsule of the present invention is preferably lyophilized to improve preservation stability. The lyophilization is preferably carried out, for example, after freezing, preferably quick-freezing, at about -20°C or less. The quick-freezing is preferably carried out using a cooling medium such as liquid nitrogen, dry ice-alcohol (e.g., ethanol, methanol, propanol, etc.), or dry ice-acetone, which is capable of cooling to -70°C or less, and more preferably -80°C or less. Liquid nitrogen is particularly preferable.

Water can be used as a medium for the heat-resistant bio-nanocapsules before the freezing. Preferably, a buffer solution is used. Examples of buffer solutions include citrate buffer, phosphate buffer, succinate buffer, acetate buffer, and like buffer solutions. Phosphate buffer solutions are preferable. Such buffer solutions may contain salts such as NaCl.

The pH of the buffer solution is preferably in the range of about 6 to about 8, and more preferably about 6.5 to about 7.5.

An excipient (a stabilizing agent) is preferably used when lyophilizing the bio-nanocapsules. Examples of such stabilizing agents include monosaccharides, disaccharides, oligosaccharides, sugar alcohols, glycol, polyhydric alcohols, and the like. Specific examples thereof include glucose, mannose, galactose, sucrose, trehalose, glycerol, erythritol, arabitol, xylitol, sorbitol, mannitol, propylene glycol, polyethylene glycol, and the like. Monosaccharides such as glucose and disaccharides such as sucrose are particularly preferable.

The stabilizing agent is added to the bio-nanocapsule solution before lyophilization. The stabilizing agent may be added to a concentration of about 5 to about 40%, and preferably 5 to 20% to achieve better preservation stability.

The amount of stabilizing agent is about 1 to about 30 parts by weight, and preferably about 5 to about 20 parts by weight, per 100 parts by weight of the bio-nanocapsule particles.

Lyophilization is performed according to a conventional method. Lyophilization can be advantageously carried out, for example, under vacuum for one day and night, e.g., for 16 hours or more.

The lyophilized bio-nanocapsule is preferably stored at 10°C or less, and more preferably at 4°C or less.

EXAMPLES

The present invention will be described in greater detail below using Examples; needless to say, however, the invention is not limited to these Examples.

The preparation method for each substance used in the Examples is described below.

(i) 0.2 M PMSF Solution

PMSF    1.7149 g

PMSF was diluted with an analytical-grade ethanol to make a total of 50 mL. The solution was stored at -20°C. The solution was passed through a filter (0.22 μm) before use.
If precipitation was observed, the solution was completely dissolved in an ultrasonic cleaner before use.

(ii) 10% Tween 80

Tween 80    5.0 mL

Tween 80 was diluted with MilliQ water to make a total of 50 mL. The solution was stored and used at 4°C. The solution was passed through a filter (0.22 μm) before use.

(iii) Buffer A (w/o Tween 80)

| | | |
|---|---|---|
| Urea (for biochemistry, Sigma 32-0291-8) | 7.5 M | 450.45 g |
| TRIZMA BASE | 0.1 M | 12.11g |
| $NaH_2PO_4$-$2H_2O$ | 50 mM | 7.2 g |
| EDTA-2Na | 15 mM | 5.58 g |

MilliQ water was added to the above components to make a total of 0.9 L, after which the mixture was adjusted to a pH of 7.4 with 1N HCl and then diluted to make a total of 1 L. The resulting buffer was stored at 4°C and used at 4°C. The buffer was passed through a filter (0.22 μm) before use.
PMSF (concentration when used: 2 mM) was also added as a protease inhibitor before use.

(iv) PBS (x 10)

| | |
|---|---|
| NaCl | 80 g |
| $Na_2HPO_4$-$12H_2O$ | 28.9 g |
| KCl | 2 g |
| $KH_2PO_4$ | 2 g |

The above components were diluted with MilliQ water to make a total of 1 L.

(v) 0.2 M $Na_2HPO_4$

$Na_2HPO_4 \cdot 12H_2O$    35.82 g

The above component was diluted with MilliQ water to make a total of 0.5 L.

(vi) 0.2 M $NaH_2PO_4$

$NaH_2PO_4 \cdot 2H_2O$    15. 6 g

The above component was diluted with MilliQ water to make a total of 0.5 L.

(vii) 0.1 M Phosphate Buffer (pH 7.2)

| 0.2 M Na$_2$HPO$_4$ | 72 mL |
|---|---|
| 0.2 M NaH$_2$PO$_4$ | 28 mL |

The above components were diluted with MilliQ water to make a total of 200 mL.

(viii) Buffer A (+ Tween 80), Prepared Before Use (Buffer for Cell Disruption)

| Buffer A (w/o Tween 80) | 500 mL |
|---|---|
| 10% Tween 80 0.1% | 5 mL |

Before use, PMSF (concentration when used: 2mM) and APMSF (concentration when used: 100 μg/mL) were added as protease inhibitors. The solution was passed through a filter (0.22 μm) before use.

(ix) PBS, 1 mM EDTA (Buffer for Dialysis)

| PBS (x 10) | | 100 mL |
|---|---|---|
| EDTA-2Na | 1 mM | 0.37 g |

The above components were diluted with MilliQ water to make a total of 1 L.
The buffer was passed through a filter (0.22 μm) before use.

(x) 10 mM Phosphate Buffer pH 7.2 (Buffer for Sulfate-Cellulofine)

0.1 M phosphate buffer (PH 7.2) 10 mM 100 mL
The above component was diluted with MilliQ water to make a total of 1 L.
The solution was passed through a filter (0.22 μm) to remove air before use.

(xi) 10 mM Phosphate Buffer pH 7.2 + 2 M NaCl (Elution Buffer for Sulfate-Cellulofine)

| 0.1 M Phosphate Buffer (PH 7.2) 10 mM | 100 mL |
|---|---|
| NaCl 2 M | 116.88 g |

The above components were diluted with MilliQ water to make a total of 1 L.
The buffer was passed through a filter (0.22 μm) to remove air before use.

(xii) PBS, 1 mM EDTA (Buffer for Gel Filtration)

| PBS (x 10) | | 100 mL |
|---|---|---|
| EDTA-2Na | 1 mM | 0.37 g |

The above components were diluted with MilliQ water to make a total of 1 L.

The buffer was passed through a filter (0.22 μm) to remove air before use.

Example 1

HBsAg particles (bio-nanocapsules) were purified in accordance with the procedures described below.

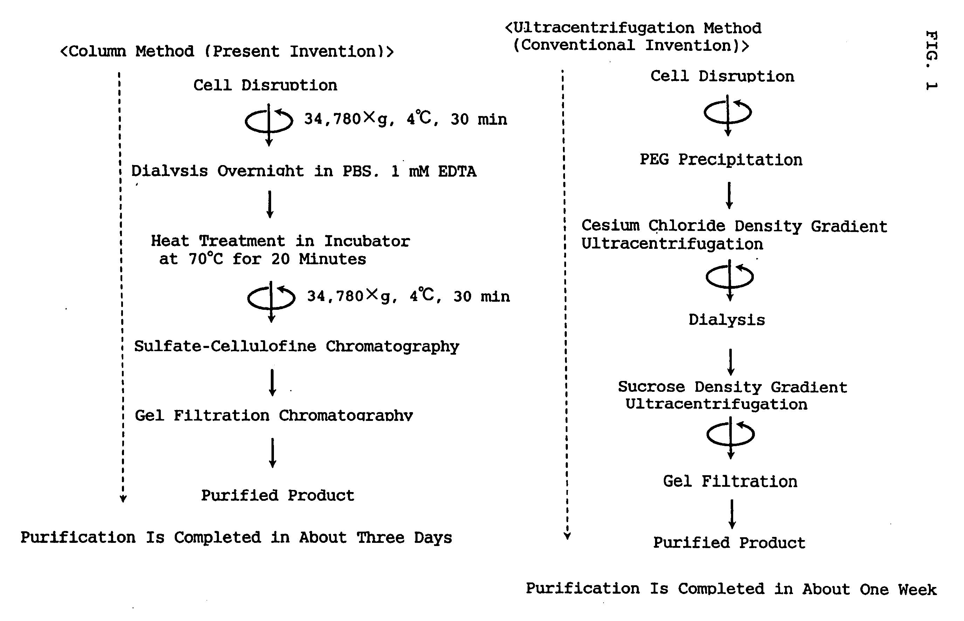

FIG. 1 shows the outline of the purification method.

(1) Cell Disruption

HBsAg particles to be disrupted were L protein particles.
The container, as well as the buffer and beads to be added were cooled to 4°C before use. 15 to 25 g of cells (corresponding to about 1 L of culture) stored at -80°C were suspended in 160 mL of Buffer A (+ Tween 80 + PMSF 4 mM + APMSF 100 μg/mL) using a pipette. The suspension was added together with 175 mL of glass beads (diameter: 0.5 mm) to a container for a BEAD-BEATER (volume: 350 mL, BioSpec Products, Inc.), after which the suspension was disrupted in ice for 2 minutes, cooled for 1 minute, disrupted for 2 minutes, cooled for 1 minute, and disrupted for 1 minute, and then cooled until the heat was removed from the container. After collection of the supernatant, the beads were washed with a small amount (about 40 mL) of Buffer A to collect the lysate, and this procedure was repeated twice. The collected lysate was centrifuged (34,780 x g, 30 min, 4°C), and then the supernatant was collected.

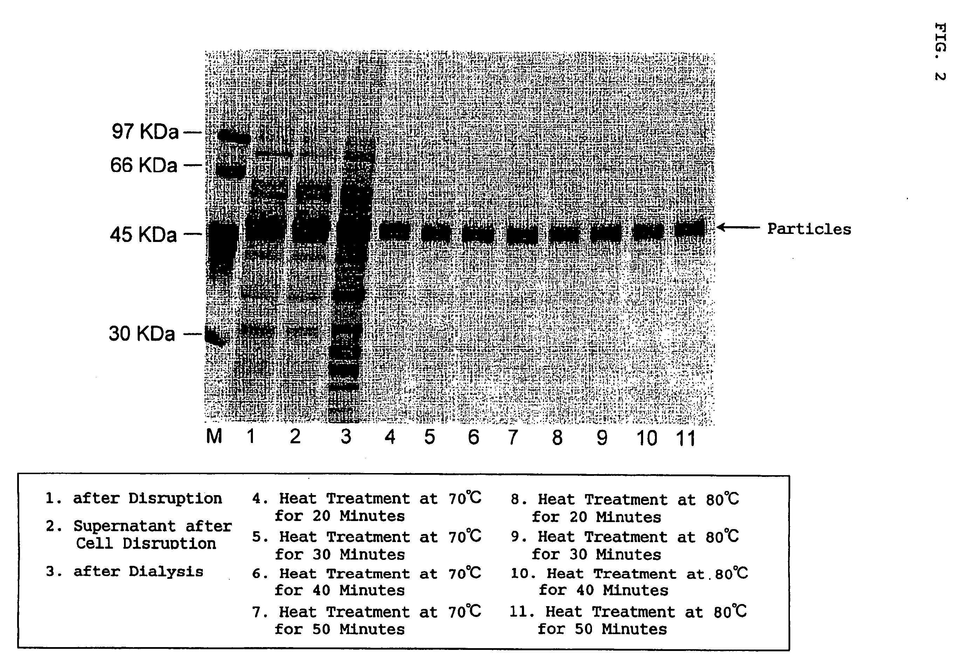

FIG. 2 shows the results of SDS-PAGE performed after cell disruption and on the supernatant.

(2) Dialysis

The sample was dialyzed against about a 20-fold volume of the external solution (PBS, 1 mM EDTA) in a low-temperature room for 1 hour, after which the external solution was replaced with an equal amount of the external solution, and then dialysis was continued for 1 hour. This procedure was repeated a total of four times (a total of 4 hours), and then dialysis was continued for 2 hours after the external solution was replaced with about a 20-fold volume of the external solution as above.
FIG. 2 shows the result of SDS-PAGE after dialysis.

(3) Heat Treatment

The dialyzed sample was dispensed in about 40 mL portions into centrifugation tubes, and heat-treated in an incubator preheated to 70°C for 20 minutes. After heat treatment, the samples were centrifuged (34,780 x g, 30 min, 4°C) and the supernatants were collected.
Similarly, each sample was heat-treated at 70°C for 30, 40, or 50 minutes, or heat-treated at 80°C for 20, 30, 40, or 50 minutes, and measured for the amount of protein (mg) and amount of antigen (unit) before and after the heat treatment. The results are shown in Table 1. Fig. 2 shows the results of SDS-PAGE after heat treatment under these conditions.
[Table 1]

| °C | min. | Amount of Protein (mg) | | Amount of Antigen (Unit) | | D/C B/A |
| | | A: Before Heat Treatment | B: After Heat Treatment | C: Before Heat Treatment | D: After Heat Treatment | |
|---|---|---|---|---|---|---|
| 70 | 20 | 50.3 | 13.4 | 9120 | 8265.6 | 3.4 |
| | 30 | 50.3 | 11.6 | 9120 | 9688 | 4.6 |
| | 40 | 50.3 | 12.7 | 9120 | 7789.6 | 3.4 |
| | 50 | 50.3 | 13.0 | 9120 | 9900.8 | 4.2 |
| 80 | 20 | 50.3 | 13.4 | 9120 | 8136.8 | 3.3 |
| | 30 | 50.3 | 8.9 | 9120 | 7263.2 | 4.5 |
| | 40 | 50.3 | 13.1 | 9120 | 8164.8 | 3.4 |
| | 50 | 50.3 | 11.7 | 9120 | 6720 | 3.2 |

(4) Sulfate-Cellulofine Chromatography

An AKTA System (Amersham Biosciences Corp.) was used.

The heat-treated samples were passed through a 0.45 μm filter, and then added to a cellulofine sulfate column (column diameter: 1.6 x 20 cm; medium: CHISSO CORP.; flow rate: 4 mL/min) equilibrated with 10 mM phosphate buffer pH 7.2 + 0.15 M NaCl. Unadsorbed fractions were washed with a volume of buffer double the volume of the column, eluted stepwise with 10 mM phosphate buffer pH 7.2 + 1 M NaCl, and fractionated into 4 mL portions. The eluate was measured for absorption at 280 nm and 260 nm. The amount of HBs antigen in each fraction was examined with IMx (ABBOTT JAPAN CO., LTD), and fractions containing HBs antigen were collected.

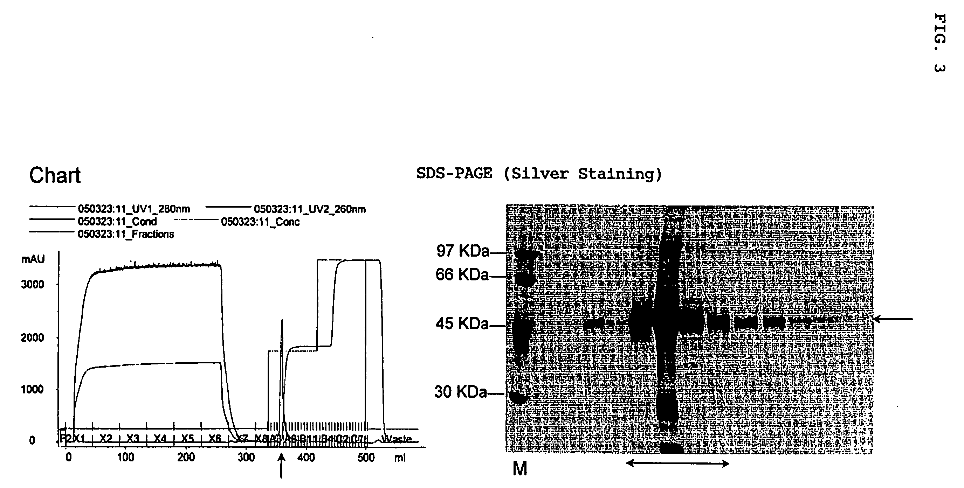

FIG. 3 shows the results of Sulfate-Cellulofine chromatography. The fraction indicated by the arrow shown in the chart of FIG. 3 was collected and passed through a gel filtration column.

The chromatography conditions were as follows.

Column: Sulfate-Cellulofine (1.6 x 20 cm)
Buffer: 10 mM Na phosphate (pH 7.2)
Elution buffer: 10 mM Na phosphate (pH 7.2), 2 M NaCl
Flow rate: 4 mL/min
Gradient: 0.15 → 0.5 M NaCl (step)
0.5 → 2 M NaCl (step)
Fraction: 5 mL/tube

(5) Ultrafiltration

The sample after Sulfate-Cellulofine chromatography was concentrated (Amicon Ultra; NMWL 100K, MILLIPORE) to 5% or less of the volume of the gel filtration column.

(6) Gel Filtration Chromatography

An AKTA System (Amersham Biosciences Corp.) was used.

The concentrated samples were passed through a 0.45 μm filter, added to a gel filtration column (column diameter: 1.6 x 60 cm; medium: Sephacryl S-500-HR; Amersham Biosciences Corp.; flow rate: 0.5 mL/min) equilibrated with PBS and 1 mM EDTA, eluted with the same buffer, and fractionated into 5 mL portions. The eluate was measured for absorption at 280 nm and 260 nm. The amount of HBs antigen in each fraction was examined with IMx (ABBOTT JAPAN CO., LTD), and also considering the results of SDS-PAGE, fractions containing HBs antigen were collected.

The chromatography conditions were as shown below.

Column: Sephacryl S-500 HR (1.6 x 70 cm)
Buffer: PBS, 1 mM EDTA
Flow rate: 0.5 mL/min
Fraction: 5 mL/tube

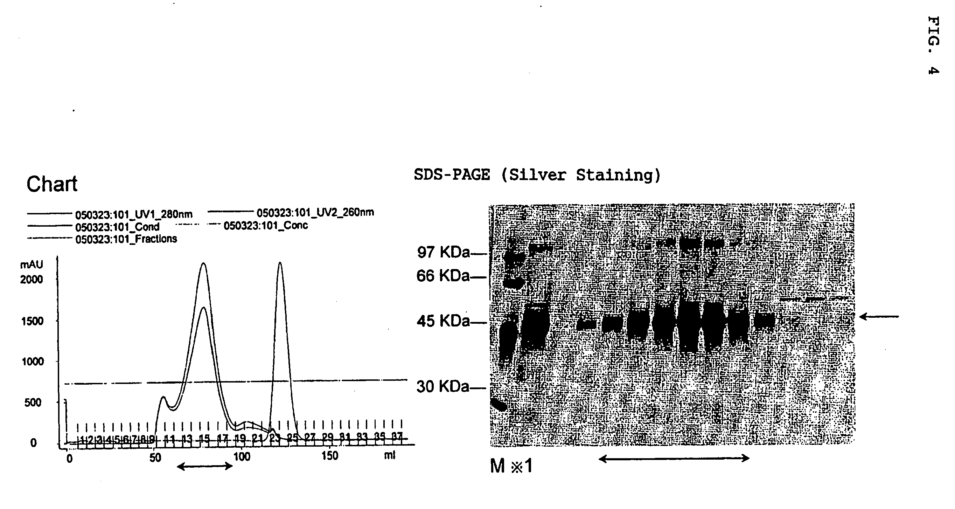

Fig. 4 shows the results of gel filtration chromatography.

(7) Ultrafiltration and Filter Sterilization

The samples obtained after gel filtration were concentrated to a protein concentration of 0.2 mg/mL or more (Amicon Ultra; NMWL 100K, MILLIPORE). The concentrated samples of the fractions indicated by the arrow shown in the chart of FIG. 4 were collected and sterilized with a 0.45 μm filter for use as purified samples.

(8) Results

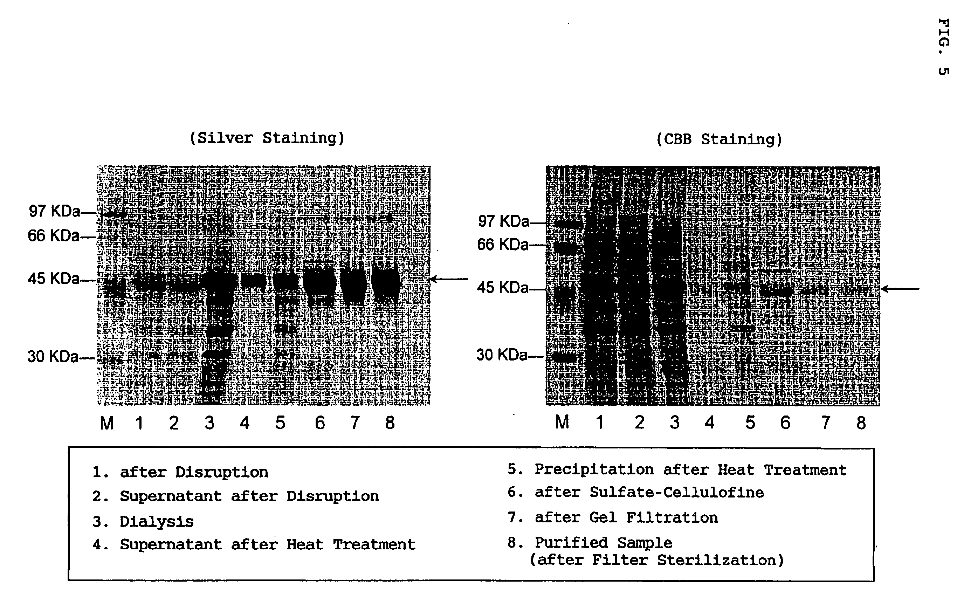

Table 2 shows the results of purification, and FIG. 5 shows the results of SDS-PAGE throughout all the steps. As shown in FIG. 5, two types of staining methods were employed: silver staining makes it easier to visualize particles, and CBB staining makes it easier to visualize contaminating proteins.

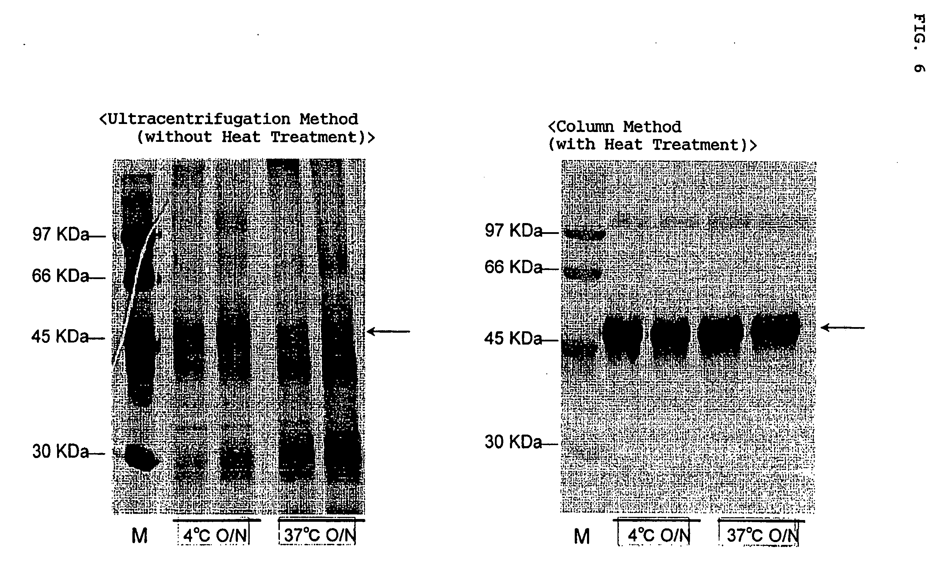

In addition, in order to evaluate the preservation stability of the particles, the fractions obtained after gel filtration were allowed to stand overnight at 4°C or 37°C, and the decomposition thereof by protease was examined. The results are shown in FIG. 6. The results in FIG. 6 show that decomposition proceeded in the samples not subjected to heat treatment, whereas the heat-treated samples were not decomposed, revealing that the purification method of the invention is effective in obtaining nanocapsules resistant to decomposition by protease.

Table 3 shows comparisons of the average values for the method of the invention and a conventional method.

[Table 2]

| Step | Amount of Protein (mg) | *1 Total Amount of Antigen (Unit) | *2 Recovery (%) | Specific Activity (Unit/mg) |
|---|---|---|---|---|
| After Disruption | 2775.4 | 170931.6 | 100 | 61.6 |
| Supernatant After Disruption | 1488.3 | 154895.8 | 91 | 104.1 |
| After Dialysis | 1200.1 | *3 174272.0 | *3 102 | 145.2 |
| Supernatant After Heat Treatment | 139.7 | 106529.2 | 62 | 762.6 |
| After Cellulofine Sulfate | 17.6 | 27108.0 | 16 | 1543.6 |
| After Gel Filtration (Purified Samples) | 8.1 | 27204.0 | 16 | 3361.0 |

*1 Equation for calculating the total amount of antigen

Total amount of antigen (Unit) = (amount of samples: mL) x (IMx RATE value) x (dilution factor of IMx)/1000

*2 Equation for calculating the recovery

Recovery (%) = (total amount of antigen in each step)/(total amount of antigen after disruption) x 100

*3 The value after dialysis is higher than the value for the supernatant after disruption because the amount of sample increased during dialysis, and is therefore within the margins of error.

[Table 3]

|  | Ultracentrifugation Method | Column Method |
|---|---|---|
| Recovery (%) | 1.8 | 15.5 |
| Specific Activity (Unit/mg) | 1971 | 3930 |

Example 2

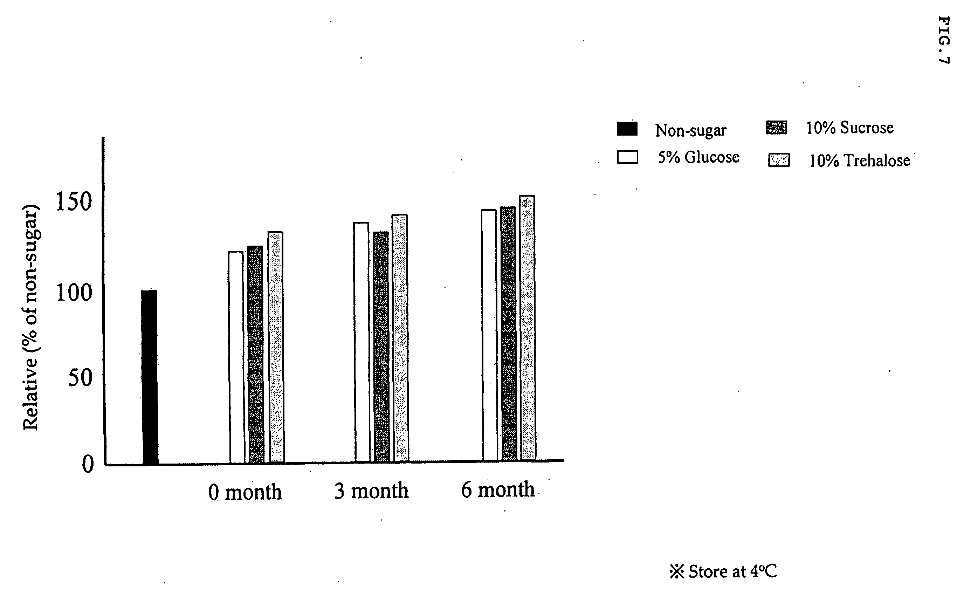

Purified bio-nanocapsules (concentration: 500 $\mu$g/mL) were dispensed into 1.5 mL tubes in 400 $\mu$l portions, and glucose was added thereto to give a final concentration of 5 wt%. The 1.5 mL tubes were then immersed in liquid nitrogen prepared beforehand and rapidly frozen. After confirming that the bio-nanocapsules in the tubes were sufficiently frozen, they were dried in a vacuum dryer for a day. The dried samples were stored at 4°C.
Moreover, sucrose and trehalose were each added to the solution instead of glucose to give a final concentration of 10 wt%, and these bio-nanocapsules were similarly lyophilized.
The lyophilized products and the control lyophilized products containing no saccharide were lyophilized at 4°C. The results are shown in FIG. 7.
The results of FIG. 7 clarify that the addition of saccharides enhances the preservation stability of the lyophilized products.

## Claims

1. A method for purifying a capsule composed mainly of a hepatitis B virus surface antigen (HBsAg) protein produced by using a eukaryotic cell, the method comprising heat-treating a eukaryotic cell lysate at 65°C to 90°C to remove contaminant proteins by precipitation, and then subjecting the resulting product to affinity chromatography and/or gel filtration chromatography.

2. A method according to claim 1, wherein the affinity chromatography is sulfate-cellulofine chromatography using a sulfate group-containing medium.

3. A method according to claim 1, wherein the affinity chromatography is antibody affinity chromatography using a medium having an anti-HBsAg antibody bound thereto, the anti-HBsAg antibody being produced by using HBsAg particles as an antigen.

4. A method according to claim 1, wherein the affinity chromatography is chromatography using hydroxyapatite as a medium.

## Patentansprüche

1. Verfahren zur Aufreinigung einer Kapsel, die hauptsächlich aus einem Hepatitis B-Virus-Oberflächenantigen (HBsAg)-Protein zusammengesetzt ist, welches durch Verwendung einer eukaryotischen Zellen hergestellt wird, wobei das Verfahren die Hitzebehandlung eines eukaryotischen Zelllysats bei 65°C bis 90°C, um verunreinigende Proteine durch Präzipitation zu entfernen, umfasst, und dann Unterziehen des erhaltenen Produkts einer Affinitätschromatographie und/oder Gelfiltrationschromatographie.

2. Verfahren nach Anspruch 1, wobei die Affinitätschromatographie Sulfat-Cellulofine-Chromatographie unter Verwendung eines Sulfatgruppenenthaltenden Mediums ist.

3. Verfahren nach Anspruch 1, wobei die Affinitätschromatographie Antikörper-Affinitätschromatographie unter Verwendung eines Mediums, das einen anti-HBsAg-Antikörper daran gebunden aufweist, ist, wobei der anti-HBsAg-Antikörper durch Verwendung von HBsAg-Teilchen als ein Antigen hergestellt wird.

4. Verfahren nach Anspruch 1, wobei die Affinitätschromatographie Chromatographie unter Verwendung von Hydroxyapatit als Medium ist.


**Revendications**

1. Procédé de purification d'une capsule composée principalement d'un antigène de la surface du virus de l'hépatite B (Ag HBs) produite en utilisant une cellule eucaryote, le procédé comprenant un traitement à la chaleur d'un lysat de cellules eucaryotes à 65 °C à 90 °C pour éliminer les protéines contaminantes par précipitation, et ensuite la soumission du produit résultant à une chromatographie d'affinité et/ou une chromatographie de filtration sur gel.

2. Procédé selon la revendication 1, dans lequel la chromatographie d'affinité est une chromatographie sur sulfate-cellulofine en utilisant un milieu contenant des groupes sulfate.

3. Procédé selon la revendication 1, dans lequel la chromatographie d'affinité est une chromatographie d'affinité pour anticorps utilisant un milieu comportant un anticorps anti-Ag HBs qui y est lié, l'anticorps anti-Ag HBs étant produit en utilisant des particules d'Ag HBs en tant qu'antigène.

4. Procédé selon la revendication 1, dans lequel la chromatographie d'affinité est une chromatographie utilisant de l'hydroxyapatite comme milieu.

FIG. 1

EP 1 985 711 B1

<Column Method (Present Invention)>

<Ultracentrifugation Method
(Conventional Invention)>

Cell Disruption

34,780×g, 4°C, 30 min

Dialysis Overnight in PBS, 1 mM EDTA

Heat Treatment in Incubator
at 70°C for 20 Minutes

34,780×g, 4°C, 30 min

Sulfate-Cellulofine Chromatography

Gel Filtration Chromatography

Purified Product

Purification Is Completed in About Three Days

Cell Disruption

PEG Precipitation

Cesium Chloride Density Gradient
Ultracentrifugation

Dialysis

Sucrose Density Gradient
Ultracentrifugation

Gel Filtration

Purified Product

Purification Is Completed in About One Week

FIG. 2

FIG. 3

SDS-PAGE (Silver Staining)

Chart

97 KDa
66 KDa
45 KDa
30 KDa

M

FIG. 4

FIG. 5

EP 1 985 711 B1

(Silver Staining)　　　　　　　　　　　　(CBB Staining)

97 KDa—
66 KDa—
45 KDa—
30 KDa—
M 1 2 3 4 5 6 7 8

97 KDa—
66 KDa—
45 KDa—
30 KDa—
M 1 2 3 4 5 6 7 8

1. after Disruption
2. Supernatant after Disruption
3. Dialysis
4. Supernatant after Heat Treatment

5. Precipitation after Heat Treatment
6. after Sulfate-Cellulofine
7. after Gel Filtration
8. Purified Sample
(after Filter Sterilization)

FIG. 6

FIG. 7

EP 1 985 711 B1

※ Store at 4ºC

**EP 1 985 711 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004002313 A
- EP 0135435 A2
- GB 1511082 A
- WO 0164930 A
- WO 03082330 A
- WO 03082344 A
- JP 2001316298 A
- JP 5252976 A
- JP 5076370 A
- JP 07714026 B
- JP 2006035666 A

**Non-patent literature cited in the description**

- *J. Biol. Chem.,* 1992, vol. 267 (3), 1953-1961
- **LEE, S.-O. et al.** *Korean J. of Immunology,* November 1987, vol. 9 (2), 233-239
- *Vaccine,* 30 April 2001, vol. 19 (23-24), 3154-63
- *J. Virol.,* 2002, vol. 76 (19), 10060-10063
- *Methods in Enzymology,* 1987, vol. 154 (350), 367-382
- *METHODS IN ENZYMOLOGY,* 1983, vol. 100, 468
- *Nucleic Acids Res.,* 1984, vol. 12, 9441
- *J. Am. Chem. Soc.,* 1967, vol. 89, 4801
- *J. AM. CHEM. SOC.,* 1969, vol. 91, 3350
- *Science,* 1968, vol. 150, 178
- *Tetrahedron Lett.,* 1981, vol. 22, 1859
- Virus Inactivation in Blood and Blood Products. WHO Global Blood Safety Initiative report, 1992, 371-398